# EUROPEAN PATENT SPECIFICATION

(11) **EP 2 953 524 B1**
(45) Date of publication and mention of the grant of the patent: **01.08.2018**
(21) Application number: 14704880.5
(22) Date of filing: 06.02.2014
(51) Int. Cl.: A61B 5/00, G01N 33/50

(54) **SYSTEMS AND METHODS FOR EARLY DISEASE DETECTION AND REAL-TIME DISEASE MONITORING**
SYSTEME UND VERFAHREN FÜR FRÜHE KRANKHEITSERKENNUNG UND ECHTZEITÜBERWACHUNG VON KRANKHEITEN
SYSTÈMES ET PROCÉDÉS POUR LA DÉTECTION DE MALADIE PRÉCOCE ET LA SURVEILLANCE DE MALADIE EN TEMPS RÉEL

(30) Priority: 06.02.2013 US 201361850028 P
(43) Date of publication of application: 16.12.2015
(73) Proprietor: Freenome Holdings Inc., South San Francisco, CA 94080 (US)
(72) Inventor: ROBERTS, Charles Edward Selkirk, South San Francisco CA94080 (US)
(74) Representative: Gibson, Mark
(86) International application number: PCT/GB2014/050353
(87) International publication number: WO 2014/122467

(56) References cited:
- GB-A- 2 441 078
- US-A1- 2009 209 904
- CHENEY C ET AL: "In vivo real-time ethanol vapor detection in the interstitial fluid of a Wistar rat using piezoresistive microcantilevers", APPLIED PHYSICS LETTERS, AMERICAN INSTITUTE OF PHYSICS, US, vol. 90, no. 1, 2 January 2007 (2007-01-02), pages 13901-013901, XP012093401, ISSN: 0003-6951, DOI: 10.1063/1.2400104
- Oxford Nanopore Technologies: "For customers - About us - Oxford Nanopore Technologies", , 24 March 2014 (2014-03-24), XP055109689, Retrieved from the Internet: URL:https://www.nanoporetech.com/about-us/ for-customers [retrieved on 2014-03-24]

## Description

### FIELD OF THE INVENTION

The invention generally relates to the field of medical device detection systems for detecting biomolecules selected from DNA or RNA indicative of disease. More specifically, the invention relates to a personal wearable or implantable detection device for detecting circulating or excreted biomolecules selected from DNA or RNA that permit the early detection and post-diagnosis monitoring of disease such as cancer.

### BACKGROUND OF THE INVENTION

It is generally accepted that early detection of disease provides the best opportunities for treatment and long term survival. In the case of cancer, technology for diagnosis and treatment of cancer is mature and cancer mortality can be reduced, especially if detected earlier. Since 1991, death rates have decreased by more than 40% for prostate cancer, and decreases of more than 30% for colon cancer, breast cancer in women, and lung cancer in men are attributed to improvements in early detection and treatment. (Cancer Statistics, 2013, R. Siegel, American Cancer Society, Atlanta, GA, 2013). Similarly, with respect to infectious diseases, early detection provides an opportunity for prompt treatment to prevent onset of more severe symptoms and also to contain spread of the disease.

Methods to personalize diagnosis of a specific tumor subtype are used to tailor treatment regimes to an individual and find the optimal treatment as early as possible. There are two components of early detection efforts: early diagnosis and screening. Early diagnosis relies on patient education to increase awareness of symptoms and screening requires a systematic testing in an otherwise asymptomatic population. Even with proper education of symptoms, by the time symptoms arise and disease is detected, it has advanced to such a state that therapies may be less effective than if the disease is detected at a pre-symptomatic early cellular stage. Current early detection and screening methods can still be seen as ad hoc blunt tools for early detection. Despite the arsenal of diagnostic tools available, lack of patient education, and infrequent testing due to cost or patient convenience is the reality facing early detection efforts. Even monitoring remission or disease progression in patients with a previous diagnosis presents obstacles to regular testing, and even more so for patients who are otherwise healthy.

Current trends in cancer treatment focus on preemptive cancer vaccines that engage an individual's immune system to monitor onset of tumor growth while a tumor is still tissue-localized at a small cell number. At early cellular stages, even the normal human immune system can often destroy and clear aberrant cell growth without the subject being aware, similar to the manner in which negative feedback loops maintain a state of homeostasis in many organ systems of the body, in a phenomenon known as the 'elimination' stage of 'immune-editing', that is, when the immune system successfully eliminates the malignancy at the stage of small numbers of cells (Dunn GP, Bruce AT, Ikeda H, Old LJ, Schreiber RD. Cancer immune-editing: from immune-surveillance to tumor escape. Nat Immunol. 2002 Nov;3(11):991-8). Vaccines also treat the condition, but they ideally need to act at such an early stage where the disease is at a localized cellular level to engage the immune system to destroy nascent tumor development before growth and metastasis.

The presence of high levels of circulating DNA in blood of tumor patients are thought to result from apoptosis and necrosis of tumor cells, or release of intact cells into the bloodstream and their subsequent lysis. Correlations between the occurrence of cell-free DNA in blood of tumor patients and malignancy of their disease have also been reported (Gormally E, et al., Mutat Res, 635:105-17 (2007); Fleischhacker M, et al. Biochim Biophys Acta 1775:181-232(2007); Jahr S et al., Cancer Res 61:1659-65 (2001); Chen X, et al., Clin Cancer Res 5:2297-303 (1999); Chun FK, et al. BJU Int 98:544-8 (2001); Schwarzenbach, H. et al., Clin Cancer Res,15; 1032 (2009)).

Methods are needed that enable the early diagnosis of the presence of cancer at early cellular stages in individuals, who are not known to have the disease, at risk of acquiring a disease, or to follow individuals who have recurrent disease using circulating biomolecules such as circulating cell-free DNA. One object of the present invention is to provide a detector device to facilitate the detection of disease-specific (e.g., pathogen-specific or tumor-specific) markers, e.g., biomolecules selected from RNA or DNA in a subject, including a human.

Also what is needed are devices for use with these methods such as highly sensitive on-body wearable, or in-body implanted devices which have the capacity to achieve real-time monitoring of the patient and detect changes in normal levels of biomolecule markers and presence of aberrant biomolecule markers of disease to permit early action with treatment while the disease is at nascent stages.

### SUMMARY OF THE INVENTION

Devices are provided that permit the early detection of biomolecule markers selected from DNA or RNA that indicate the presence of a disease such as cancer, or are associated with the development of a disease.

Advances in nanotechnology and miniaturization enable the production of a wearable or implantable device as part of a system for short term or long term monitoring of biomolecules that identify onset of a disease sooner than diagnostic screens that occur through routine physician visits. The frequency of occurrence of a biomolecule detected in a biological fluid in a closed system such as the circulation is used with the detector device herein as an indicator of disease and disease progression with and without treatment. Generally the detector device is one of three units within a system : 1) a biomolecule detector, 2) a receiver-relay and 3) a processor.

According to a first aspect of the invention, there is provided a detector device for detecting the presence of one or more biomolecule markers selected from DNA or RNA in a biological fluid in a subject comprising:
a) detection means in fluid communication with a biological fluid capable of obtaining information regarding the identity and concentration of a biomolecule, wherein the detector includes a sequencing module, wherein the sequencing module is a nanopore detection device comprising one or more nanopores for detecting and sequencing DNA or RNA,
b) a power supply, and
c) means to transmit the information to a receiver-relay unit.

In one embodiment, the nanopores are biological, solid-state or hybrid pores that permit the detection and sequencing of nucleic acid to provide real-time DNA sequencing of nucleic acids in a biological fluid.

In one embodiment, the solid-state nanopores are made of synthetic materials such as silicon nitride or graphene.

In one embodiment, the detector comprises an array of nanopores for multiplex evaluation of biomolecules.

In one embodiment, the detector is implantable located at a surgically created arterio-venous fistula or shunt, between the arterial and venous systems.

In one embodiment, the detector includes an array of nanopores for multiplex detection of nucleic acid bases and linear nucleic acid sequencing.

In one embodiment, the detector is arranged in such a manner as to permit flow of a biological fluid through the nanopore array to permit detection and evaluation of the biomolecules without substantially affecting the flow of the biological fluid on its proper course.

In one embodiment, the array of nucleic acid base-detecting nanopores permits sequencing of nucleic acid present in the biological fluid to evaluate the identity of, and characteristics of the nucleic acid that indicates disease.

In one embodiment, a biological protective matrix layer covers the surface of the detector device.

In one embodiment, data transmission to the receiver-relay is continuous or periodic and can be controlled by receiving instruction signals from the receiver-relay.

In one embodiment, the device is implantable or wearable in a host.

In one embodiment, the device is implantable in a blood vessel or a blood vessel bypass graft.

In one embodiment, the device is in fluid communication with the lymphatic system.

In one embodiment, the device further comprises means for monitoring, and a feedback loop to control directly, and/or signal the patency of the feeding vessel/system and biological fluid flow to the device and resultant reliable access to biomolecules.

### BRIEF DESCRIPTION OF THE DRAWINGS

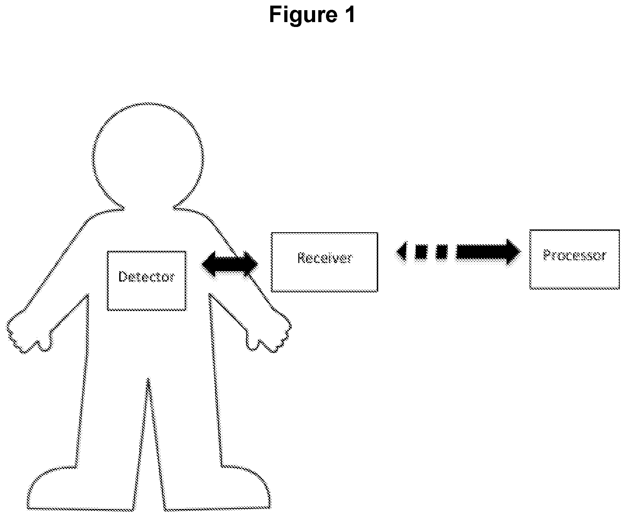

Figure 1 provides a block diagram showing the general components of the early disease detection system.

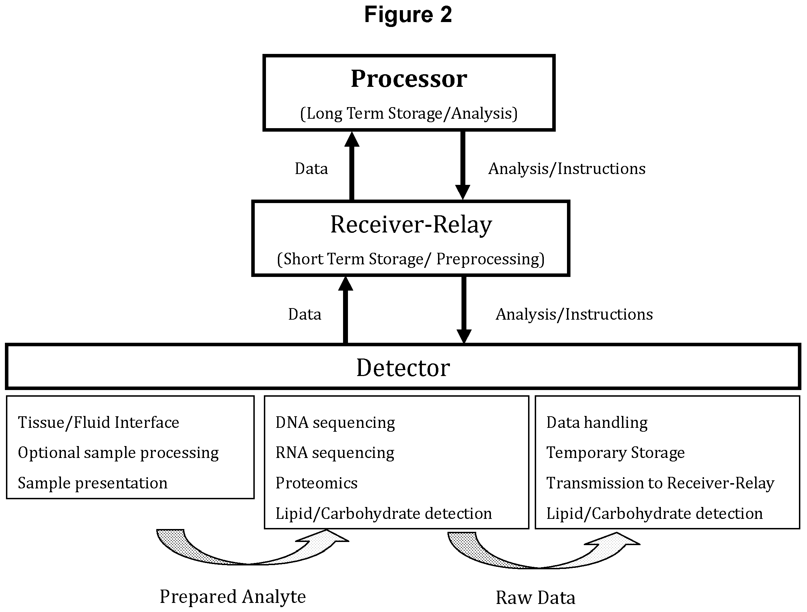

Figure 2 provides a block diagram showing a more detailed illustration of the components of the system and interaction of the parts.
Figure 3 provides a schematic showing one embodiment where the detector unit is implanted as part of a blood vessel graft for monitoring of biomolecules in the blood. Arrow denotes flow of blood through the blood vessel and graft containing the detector unit. Electrical symbol denotes transmission of data from detector unit.
Figures 4A-C provides a schematic with different views showing a particular embodiment, where the detector unit is implanted as part of a blood vessel stent. Arrow denotes the flow of blood through the blood vessel. Electrical symbol denotes transmission of data from detector unit.

### DETAILED DESCRIPTION OF THE INVENTION

### DEFINITIONS

Unless defined otherwise, technical and scientific terms used herein have the same meaning as commonly understood by one of ordinary skill in the art to which this invention belongs. For purposes of describing the present invention, the following terms are defined below.

As used herein the terms, "a" or "an" may mean one or more. As used herein in conjunction with the word "comprising", the words "a" or "an" may mean one or more than one.

As used herein the term "biomolecule" refers to RNA or DNA.

As used herein the term "biological fluid" refers to fluids within, excreted by or secreted by living organisms. Exemplary biological fluids include saliva, whole blood, plasma, serum, lymph, synovial fluid, peritoneal fluid, pleural fluid, urine, sputum, semen, vaginal lavage, bone marrow, cerebrospinal cord fluid and tears.

As used herein the term "biomolecule characteristics" refers to physical and chemical characteristics of a biomolecule that allow one to distinguish between different biomolecules. Such features include nucleic acid sequence, secondary and tertiary structures, molecular weight, chemical structures and degree of structural branching.

As used herein, the term "fluid communication" refers to physical communication between a fluid and an object such that the fluid is in physical contact with, or flows through orifices of the object such that substantial surface area contact occurs between the fluid and the object.

As used herein, the terms "disease," "disorder" and "condition," describe a pathological condition in an organism including any impairment of health or any condition of abnormal function resulting from cause or condition including, but not limited to, infections, acquired conditions, genetic conditions, and characterized by identifiable symptoms.

As used herein, the terms "cancer" and "cancerous" refer to or describe the physiological condition in mammals that is typically characterized by unregulated cell growth. Examples of cancer include, but are not limited to, melanoma, carcinoma, lymphoma, blastoma, sarcoma, and leukemia or lymphoid malignancies. More particular examples of cancers include squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

As used herein, the term "hyperproliferative disease" is defined as a disease that results from a hyperproliferation of cells. Exemplary hyperproliferative diseases include, but are not limited to, cancer or autoimmune diseases. Examples include, but are not limited to, cancers, such as the cancer is melanoma, non-small cell lung, small-cell lung, lung, hepatocarcinoma, retinoblastoma, astrocytoma, glioblastoma, gum, tongue, leukemia, neuroblastoma, head, neck, breast, pancreatic, prostate, renal, bone, testicular, ovarian, mesothelioma, cervical, gastrointestinal, lymphoma, brain, colon, sarcoma or bladder cancer. The cancer may include a tumor comprised of tumor cells. In other embodiments, the hyperproliferative disease is rheumatoid arthritis, inflammatory bowel disease, osteoarthritis, leiomyomas, adenomas, lipomas, hemangiomas, fibromas, vascular occlusion, restenosis, atherosclerosis, pre-neoplastic lesions (such as adenomatous hyperplasia and prostatic intraepithelial neoplasia), carcinoma in situ, oral hairy leukoplakia, or psoriasis.

As used herein, the terms "neoplasm" or "neoplastic cells" refer to cells that multiply in an abnormal manner. Neoplasms can be classified as either benign, histoid, malignant, mixed multicentric, organoid or unicentric.

As used herein, the term "tumor" refers to a localized concentration, gathering or other organization (including but not limited to hyperproliferative cells located within a sheath (theca) or organ) of hyperproliferating (hyperproliferative) cells, including for example but not limited to neoplastic cells, whether malignant or benign, pre-cancerous and cancerous cells.

As used herein, the term "variant," "variants," "mutated," and the like, refer to proteins or peptides and/or other agents and/or compounds that differ from a reference protein, peptide or other compound. Variants in this sense are described below and elsewhere in the present disclosure in greater detail. For example, changes in the nucleic acid sequence of the variant may be silent, e.g., they may not alter the amino acids encoded by the nucleic acid sequence. Where alterations are limited to silent changes of this type a variant will encode a peptide with the same amino acid sequence as the reference peptide. Changes in the nucleic acid sequence of the variant may alter the amino acid sequence of a peptide encoded by the reference nucleic acid sequence. Such nucleic acid changes may result in amino acid substitutions, additions, deletions, fusions and truncations in the peptide encoded by the reference sequence, as discussed below. Generally, differences in amino acid sequences are limited so that the sequences of the reference and the variant are closely similar overall and, in many regions, identical. A variant and reference peptide may differ in amino acid sequence by one or more substitutions, additions, deletions, fusions and truncations, which may be present in any combination. A variant may also be a fragment of a peptide of the invention that differs from a reference peptide sequence by being shorter than the reference sequence, such as by a terminal or internal deletion. Another variant of a peptide of the invention also includes a peptide which retains essentially the same function or activity as such peptide.

As used herein, the terms "systemic" and "systemically" refer to contact with at least one system associated with the whole body, such as but not limited to the circulatory system, immune system, and lymphatic system, rather than only to a localized part of the body, such as but not limited to within a tumor.

As used herein, "patient", "individual" or "subject" includes humans and or non-human animals, including mammals. Mammals include primates, such as but not limited to humans, chimpanzees, gorillas and monkeys; domesticated animals, such as dogs, horses, cats, pigs, goats, cows; and rodents such as mice, rats, hamsters and gerbils.

### I. Target Biomolecules

The devices described herein are useful for the detection and identification of biomolecule markers, also referred to as analytes, selected from DNA or RNA that are known to be associated with disease. In some embodiments, the target marker is present in a biological fluid within or excreted or secreted by a living organism such as blood, blood plasma, serum, sweat or urine. One or more of the aforementioned targets may be characteristic of particular cells, while other targets may be associated with a particular disease or condition. In some embodiments, targets that may be detected and analyzed using the methods disclosed herein may include, but are not limited to, prognostic targets, hormone or hormone receptor targets, lymphoid targets, tumor targets, cell cycle associated targets, neural tissue and tumor targets, or cluster differentiation targets. Although some markers are normally immobilized on cell surface, these markers may be detected in a biological fluid after cell lysis and before being sequestered by biological mechanisms for excretion. In one embodiment, the target is present in a biological fluid for detection using the detector device described herein. The frequency of occurrence of a biomolecule detected in a biological fluid in a closed system such as the circulation is used with the detector device herein as an indicator of disease and disease progression with and without treatment. Sources of biomolecules include cells of the body and the corresponding transcriptomes, as well as cell-free nucleic acids.

### Cellular transcriptomes

The transcriptome is the set of all RNA molecules in the population of cells and reflects the genes that are being actively expressed at a specific point in time. In one embodiment, where the cell population is the blood cell population, the continuous interactions between blood cells and the entire body supports the use of detecting subtle changes occurring in association with injury or disease within the cells and tissues of the body. Blood may serve as an ideal source of biomarker targets for diagnostic/prognostic purposes. Different gene expression signatures exist in circulating blood cell for a number of disease states. (Liew et al., J Lab Clin Med 147(3):126-32 (2006)). Blood cells can act as sentinels of disease and that we could capitalize on this property of blood for the diagnosis/prognosis of disease (the "Sentinel Principle"). Peripheral blood is an ideal surrogate tissue as it is readily obtainable, provides a large biosensor pool in the form of gene transcripts, and response to changes in the macro- and micro-environments is detectable as alterations in the levels of these gene transcripts. Changes in blood transcriptome can occur within two weeks of disease treatment (WO 2013138497; Bloom et al., PLOS ONE, 7(10):1-13 (2012))

### Cell-Free DNA

Cell-free Nucleic Acids (cfNA) was first described 60 years ago. cfNA are nucleic acids that are no longer confined within cells but are dispersed in body fluids or in circulation. A biological fluid can provide the genetic landscape of a disease state such as cancer (primary and metastatic) and offer a way to systematically track genomic evolution. (Crowley et al, Nat. Rev. Clin. Oncol. 2013) On average, the size of cfDNA varies between small fragments of 70 to 200 base pairs and large fragments of approximately 21 kilobases. Current cfDNA measurement methods are work-intensive and expensive. Consequently, cfDNA measurements are not used during routine management of patients in clinical settings. Methylated DNA, cfRNA and circulating miRNAs are potential disease biomarkers in blood. There are two basic approaches to cfDNA analysis: quantitative analysis and analysis based on DNA-specific mutations.

In the case of cancer-associated cfNA, cfNA carries great potential in either complementing or superseding existing cancer tissue and blood biomarkers and screening for cfNA beings a viable tool in early detection and management of major cancers. Fractional concentrations of tumor-derived cfNA in plasma can be correlated with tumour size and surgical treatment. (Gormally et al. Mutation Research 635(2-3):105-17, (2007); Chan et al., Clin Chem 59(1):211-224 (2013); Czeiger et al., Am J Clin Pathol 135:264-270 (2011); Garcia-Olmo, et al., Mol Cancer 12(8):1-10 (2013). While not to be bound by a specific mechanism, it is believed that cfNA is released from tumors primarily due to necrotization, whereas the origin of non-tumourous cfNA is mostly apoptotic. One method to distinguish tumor cfNA from non-tumorous cfNA, includes detecting specific somatic DNA mutations, previously localized in the primary tumor that can be identified in the cfNA. (Benesova et al., Anal. Biochem. 433:227-234 (2013). cfNA yields are found in higher amounts in patients with malignant and benign tumours when compared to healthy subjects. For example, a tumour that weighs 100 g, which corresponds to 3 x 10¹⁰ tumour cells, may yield up to 3.3% of tumour DNA may enter the blood every day (an average of 180 ng per ml cfDNA, compared to an average of 30 ng per ml cfDNA in healthy subjects). Measuring cfDNA to predict treatment response is particularly attractive, particularly, in stage IV cancer patients (where cfDNA is better correlated to tumor presence), where biopsies are not possible or repeat sampling of primary tumour and metastatic samples is not practical or ethical.

### II. Devices

### Detector units

The biomolecule detector or detection units described herein, also referred to as a nanodetection units, are sufficiently miniaturized to provide portable or implantable monitoring of biomolecules in a biological fluid of an individual. Detector units are preferably biocompatible, and hermetically sealed except for a portal to permit physical communication of a biological fluid containing a biomolecule to be detected. The detector units are designed to permit fluid contact and/or flow through of a biological fluid. If implanted, the detector unit may be configured (for example, is configured) to permit flow through of a biological fluid along the same trajectory it conventionally flows without substantial disruption in flow. The detector device includes means to detect biomolecules in a biological fluid within, excreted by, or secreted by a living organism. In one embodiment, the means for detecting biomolecules is an array of nanopore units. In another embodiment, the means for detecting biomolecules is sequencing-by-synthesis, using such methods as silicon chip ion sequencing. In another embodiment, the means for detecting biomolecules is a lab-on-a-chip.

The "lab-on-a-chip" technology integrates one or several laboratory functions on a single miniature device of only millimeters to a few square centimeters in size. Lab-on-a-chip technologies are complementary and useful since they also analyze extremely small fluid volumes down to less than pico liters. Lab-on-a-chip devices are a subset of microelectromechanical system (MEMS) devices that are often indicated by "Micro Total Analysis Systems" (µTAS) as well and are closely related to, and overlap with, microfluidics which studies minute amounts of fluids. Other means or assays for detecting biomolecules that maybe suitably miniaturized for use in the devices described herein are contemplated for use in the detection unit.

In other embodiments, the detector units can include data storage means, power supply, transmitter/receiver for wireless signals, and a microprocessor. In one embodiment, preliminary information regarding the biomolecule identity such as nucleotide sequence, molecular weight or binding properties is stored in memory of the detector and relayed to an external receiver-relay unit in close proximity to the detector. Examples of such functions are described in U.S. Patent 5,836,889 and U.S. patent publications 2004/0199222 and 2012/0123221. The detector may optionally include a preliminary processor for screening raw data according to pre-set or modifiable settings, and packaging biomolecule information in data files for transmission for the receiver-relay. The detector may also include a receiver for receiving signals to modify the processing or data collection functions without having to remove an implanted device

In one embodiment, the detector is configured to permit the flow through of a biological fluid for evaluation. In one embodiment, the detector includes means to capture biomolecules flowing through the detector for subsequent detection and identification. One such a configuration is a double lumen implant device as described in WO1993/05730. Other such features of the device are contemplated that permit flow through of the biological fluid through the detection unit without substantially obstructing the regular flow of the biological fluid.

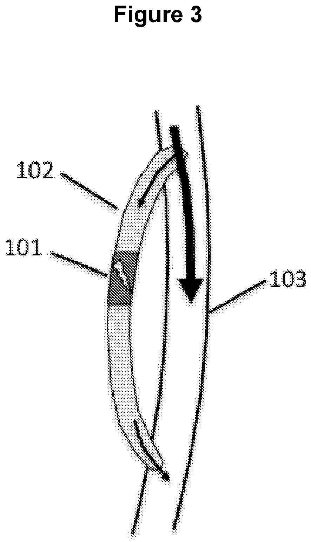

In one embodiment, the detector (101) is part of a blood vessel graft (102) similar to grafts used in blood vessel (103) bypass procedures (FIG. 3). In one embodiment, the detector is positioned in the arterial flow to measure biomolecules through the blood circulation. In one embodiment, the detector is positioned in the venous flow to measure biomolecules through the blood circulation.

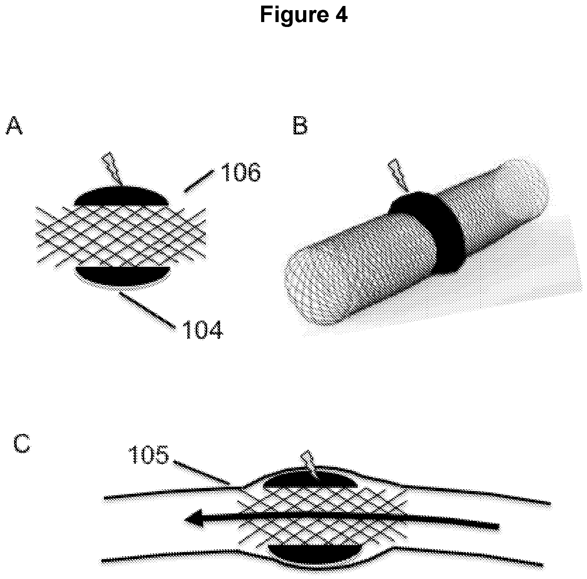

In another embodiment, the detector (104) may be positioned in a blood vessel (105) using a stent (106) similar to a stent used in endovascular procedures and applications (FIG. 4). In one embodiment, the detector/stent includes therapeutic drugs commonly used with stent applications. In another embodiment, the detector/stent includes one or more therapeutic drug compounds to minimize trauma or inflammation of the blood vessel in which the detector/stent is implanted.

In another embodiment, Vertically Aligned Carbon Nanotubes (VACNT) microfluidic devices (Roy et al, J R Soc Interface. 2010 July 6; 7(48): 1129-1133; Chen et al., Lab Chip. 2012 Sep 7;12(17):3159-67) are employed to obtain the adequate flow of biological fluid for detection of biomarkers. VACNT elements can provide nanoscale filtration and diffusion length scales for nano-bioparticle separation, without the low throughput that limits many other nanofluidic technologies. This provides unprecedented flexibility in device design that enables fluid and particle manipulation at the micro and nano-scale. Nanoporous VACNT can be integrated into patterned polymethyl siloxane (PDMS) channels using a strategy derived from standard PDMS channel fabrication and bonding techniques. This characteristic makes VACNT filtering system compatible with PDMS-microchannels solid-state nanopore networks (Tarun 2013) In one embodiment, a position of Y-filters made of VACNT forest at the front end of the microfluidic system is employed to exclude large biomolecules and cells. The application of a very low voltage with the positive electrode on the inside of the system may be used to selectively translocate negatively charged biomolecules (including but not limited to nucleic acids) across a first interface for detection using biomarker detection means such as a sensing nanopore sequencing array.

In another embodiment, the detector unit may be positioned: on the surface of the skin - for example to detect analytes in sweat; on mucous membranes - for example to detect analytes in saliva or other mucous membrane secretions; in the gastro-intestinal tract - for example to detect analytes in the gastro-intestinal tract; in the bladder or elsewhere in the genito-urinary system - for example to detect analytes in the urine; elsewhere inside the body, or inside a blood vessel, to position the detector unit in fluid communication with a biological fluid to be evaluated for biomolecules. Suitably, the detector unit may be positioned on the surface of the skin, inside the body, or inside a blood vessel to position the detector unit in fluid communication with a biological fluid to be evaluated for biomolecules.

In one embodiment, miniaturization of the detector device includes methods of microfluidic bioseparation such as MEMS and NEMS. Traditional microelectromechanical systems (MEMS) rely on photolithography which can produce feature sizes of approximately 1 micron. Nanoporous monoliths inside microfluidic channels or the fabrication of nanoscale channels with electromechanical systems (NEMS) techniques that rely on for example E-beam lithography may produce features in the nano range.

Nanopore detection units are employed to detect biomolecules in biological fluids of an individual. Examples of nanopore detection units are described for example in Gu, L-Q, et al., Nature 398:686-690, 1999; Braha, O., Chem & Biol, 4:497-505, 1997; Bayley H et al., Nature 413:226-230, 2001; Shin, S-H, et al., Angew. Chem. Int. Ed. 2003, 41(19); 3707-3709; Guan X., et al., ChemBioChem 6:1875-1881, 2005; and Braha, O. et al., ChemPhysChem 6:889-892, 2005

Engineered versions of the bacterial pore forming toxin α-hemolysin (a-HL) have been used for stochastic sensing of many classes of molecules (Bayley, H., and Cremer, P. S. (2001) Nature 413, 226-230; Shin, S.-H., et al. (2002) Angew. Chem. Int. Ed. 41, 3707-3709; and Guan, X. et al.,. (2005) ChemBioChem 6, 1875-1881). In the course of these studies, it was found that attempts to engineer α -HL to bind small organic nucleotides directly can prove taxing, with rare examples of success. Fortunately, a different strategy was discovered, which utilized non-covalently attached molecular adaptors, notably cyclodextrins (Gu, L.-Q., et al., (1999) Nature 398, 686-690), but also cyclic peptides (Sanchez-Quesada, J. et al., (2000) J. Am. Chem. Soc. 122, 11758-11766) and cucurbiturils (Braha, O., et al., (2005) ChemPhysChem 6, 889-892). Cyclodextrins become transiently lodged in the α -HL pore and produce a substantial but incomplete channel block. Organic nucleotides, which bind within the hydrophobic interiors of cyclodextrins, augment this block allowing electrophysiological detection (Gu, L.-Q., et al., (1999) Nature 398, 686-690). Nanopore detection arrays are described in US2011/0177498; US2011/0229877; US2012/0133354; WO2012/042226; WO2012/107778, and have been used for nucleic acid sequencing as described in US2012/0058468; US2012/0064599; US2012/0322679 and WO2012/164270. A single molecule of DNA can be sequenced directly using a nanopore, without the need for an intervening PCR amplification step or a chemical labelling step or the need for optical instrumentation to identify the chemical label. Commercially available nanopore nucleic acid sequencing units are developed by Oxford Nanopore (Oxford, United Kingdom). The GridION™ system and miniaturised MinION™ device are designed to provide novel qualities in molecular sensing such as real-time data streaming, improved simplicity, efficiency and scalability of workflows and direct analysis of the molecule of interest. Using the Oxford Nanopore nanopore sequencing platform, an ionic current is passed through the nanopore by setting a voltage across this membrane. If an analyte passes through the pore or near its aperture, this event creates a characteristic disruption in current. Measurement of that current makes it possible to identify the molecule in question. For example, this system can be used to distinguish between the four standard DNA bases G, A, T and C, and also modified bases. These nanopore arrays are useful for scientific applications specific for each analyte type; for example when sequencing DNA, the technology may be used for resequencing, de novo sequencing, and epigenetics. Notably, for the device disclosed herein, the Oxford Nanopore sequencing technologies such as the MinION are able to work on whole blood and other non-prepared fluid samples (suitably whole blood) as an analyte source fluid, readily facilitating placement on or in the host as disclosed herein.

In one embodiment, networks of nanopores may be developed for use in the detection devices described herein using a combined process that integrates membranes containing nanopores into microfluidic devices. This use of nanopore networks confers the advantage of decreasing noise and enabling the design of networks containing nanopores. The resulting nanopores can sense single DNA molecules at high bandwidths and with low noise due to reductions in membrane capacitance. Each step of the fabrication scheme is modular and can be independently adjusted to achieve specific functions. Thus, the assembly process is amenable to different nanopore fabrication schemes (TEM drilling, helium ion beam machining) and other materials such as graphene. The device architecture reduces capacitative noise and allows for exploiting the capabilities of low-noise amplifiers for nanopores. This approach provides a means to enable large-scale integration of solid-state nanopores with microfluidic upstream and downstream processing and permit new functions with nanopores such as complex manipulations for multidimensional analysis and parallel sensing in two and three-dimensional architectures. The present detector device uses nanopore detection units which are sufficiently miniaturized to be worn on the person or implanted within the individual such that the detection unit is in fluid communication with a biological fluid.

In one embodiment, the nanopore array permits DNA sequencing. "Strand sequencing" is a technique that passes intact DNA polymers through a protein nanopore, sequencing in real time as the DNA translocates the pore. (Oxford Nanopore Technologies, Oxford, UK) A single-stranded DNA polymer is passed through a protein nanopore, and individual DNA bases on the strand are identified in sequence as the DNA molecule passes through. This method can be used to generate read lengths of many tens of kilobases. And may be performed for example of Oxford Nanopore Technologies GridION™ system; strand-sequencing nanopore/enzyme constructs are provided in the single-use cartridge. The method may also be performed using the smaller MinION™ device. Oxford Nanopore has engineered bespoke nanopores, and data analysis algorithms are used to translate the characteristic electronic signals from stochastic sequencing into DNA sequence data.

In one embodiment, the nanopore array permits "Exonuclease sequencing" that passes individual nucleotides through a protein nanopore, aided by a processive exonuclease enzyme. "Wild type" (naturally occurring) α-hemolysin nanopore alone is not capable of differentiating DNA bases. Oxford Nanopore uses protein engineering techniques to adapt the nanopore for the detection of DNA bases. In the exonuclease method of DNA sequencing, a protein nanopore is coupled with a processive enzyme, an exonuclease. The enzyme cleaves individual DNA bases from a DNA strand. These bases enter the nanopore and undergo a binding event before passing through the pore. During this binding event, they cause characteristic disruption in current that can be used to identify the DNA bases in sequence. This method provides the identification of individual nucleoside 5'-monophosphate molecules (DNA bases) to a standard commensurate with a high resolution DNA sequencing technology.

DNA bases in solution enter the nanopore and one by one, the bases transiently bind to the cyclodextrin adapter. Each time a base passes through the pore there is a disruption in a current measured across the pore that indicates the identity of the base in the molecule sequence. RNA sequencing uses nanopores, customized using processive enzymes specific for RNA, and adapting the nanopore to distinguish RNA-specific bases, this RNA analysis system can also be integrated for use with the GridION™ platform. This system is designed to analyse the original sample RNA strand directly, rather than by undergoing conversion to cDNA. The simplification of workflow, and direct - rather than surrogate - analysis, is unique to nanopore sensing and facilitates the use of an onboard/implantable system for biomolecule detection.

In another embodiment, the nanopore array includes a sub-microsecond temporal resolution that is compatible with manufacturing methods for integration of nanopores in microfluidic devices. This provides a low-noise measurement platform that integrates a complementary metal-oxide semiconductor (CMOS) preamplifier with solid-state nanopores in thin silicon nitride membranes. This configuration provides a signal-to-noise ratio exceeding five at a bandwith of 1MHz which is adequate for these purposes.

This device centers around a low-noise current preamplifier and a high-performance solid-state nanopore. The preamplifier circuitry occupies 0.2mm² with a 0.13 micron mixed-signal CMOS process and is positioned directly inside the fluid chamber. This configuration significantly reduces parasitic capacitance. Lower noise spectral density will yield more accurate base calls, and with sufficient signal amplitudes, wider signal bandwidth can support faster translocations and higher throughput required in these detection methods.

### BioCompatibility

Biocompatibility of an implanted or on-board device is a consideration to ensure stability of the device when in fluid communication with a biological fluid. The United States Food & Drug Administration recommends the use of International Standard ISO-10993, "Biological Evaluation of Medical Devices Part 1: Evaluation and Testing" as a guidance document to ensure biocompatibility of parts of a medical device which contact a patient.

In one embodiment, a polymer encasing is used to prevent the foreign-body reaction which includes the formation of a collagenous capsule that isolates the device separating it from fluid communication with the body thereby impairing function. Polymers such as PEG and PHEMA are commonly used non-fouling or low-fouling materials and have been applied to implantable materials and devices. In another embodiment, the polymer is carboxybetaine (CBMA). CBMA is unique and has been shown to adsorb <0.3 ng/cm² proteins from 100% blood plasma or serum and is structurally similar to glycine betaine which is an endogenous solute that plays an important role in osmotic regulation. Compared to PHEMA hydrogels, a zwitterionic hydrogel prepared form CBMA monomer and CBMA cross-linker more effectively mitigates the foreign-body reaction (Zhang et al., 2013).

Other biocompatible materials are known in the art. Titanium and titanium alloys are frequently used for medical applications and may be used for the casings of implantable medical devices.

In another embodiment, a biological protective matrix layer covers the surface of the underlying detector device. In one embodiment the matrix is combined with any of the previously disclosed methods, and/other materials known in the art and preserving fluid communication with the biological fluid. In this embodiment, biocompatible matrix material such as that employed by artificial skin substitutes such as INTEGRA® (Integra LifeSciences Holdings Corp., Plainsborough, NJ) and the like, to encourage integration of the device with host tissue, and to minimize constrictive fibrosis and other host responses to the device.

In another embodiment, the biological protective layer is optionally combined with the matrix or any of the methods disclosed herein. In yet another embodiment, a biological protective layer incorporates engineered tissue which may be cellular in origin.

In one embodiment, the biological layer is a 3-dimensional layer of cultured cells, which may be denuded of immunogenic elements or may be derived from the host individual's own tissues and therefore compatible which host immunity, thereby eliciting little or no response. Methods for culturing and achieving 3-dimensional biological layer of cultured tissues are known in the art, and employed using bio-printing by such entities as Organovo Holdings Inc. (San Diego, CA), and academic institutions such as the laboratory of Dr Anthony Atala (Wake Forest School of Medicine, Winston-Salem, NC).

As described above, for implantation, the detector unit packaging must exhibit long term hermeticity (on the order of the life of the sensor). Fluid communication via feedthroughs for the biological fluid to flow through must be provided through the hermetic package that do not introduce new or unnecessary potential modes of failure. The feedthroughs will constitute a necessary material interface, but all other interfaces can and should be eliminated. In other words, the number and area of material interfaces should be minimized to reduce the potential for breach of hermeticity. The term hermetic is generally defined as meaning "airtight or impervious to air." In reality, however, all materials are, to a greater or lesser extent, permeable, and hence specifications must define acceptable levels of hermeticity. An acceptable level of hermeticity for a pressure sensor, for example, is therefore a rate of fluid ingress or egress that changes the pressure in the internal reference volume (pressure chamber) by an amount preferably less than 10 percent of the external pressure being sensed, more preferably less than 5 percent, and most preferably less than 1 percent over the accumulated time over which the measurements will be taken. In many biological applications, for example, an acceptable pressure change in the pressure chamber is on the order of 1.5 mm Hg/year. It is to be understood that that the present invention is not limited only to hermetic sensors or sensing devices that sense pressure, but may include any sensor or device that employs a hermetic chamber or cavity. The materials selected must be compatible with the processes used to fabricate the package as well as sufficiently robust to resist deleterious corrosion and biocompatible to minimize the body's immune response. Finally, the packaging should be amenable to batch fabrication. Examples of such packaging methods are described in U.S. Patent Publication 20060174712. In one embodiment the detection units include a power supply to provide power for function of the detection unit. Energy harvesting devices for implants or methods of externally charging an implanted medical device are described for example in WO2010/005915 and EP116820.

A variety of hardware is available in the art for use in the devices described herein. In one embodiment, the implantable or wearable detector device includes a power supply. In one embodiment, the power supply includes one or both of a primary battery and a rechargeable battery.

In another embodiment, the rechargeable battery is a body energy harvesting battery where power is obtained from the body and stored in the battery. The human body produces a considerable about of energy and several microsystems can harvest the energy to power IMDs. In one specific embodiment, a piezoelectric diaphragm transduces pressure variations in blood vessels into electrical energy. In another embodiment, implantable microbiofuel cells harvest chemical energy from glucose.

In another embodiment, the rechargeable battery is charged through an external control unit using electromagnetic waves or mechanical waves.

In one embodiment where electromagnetic waves are used to charge the battery, charging can occur by inductive coupling or low-power frequency.

In an embodiment using inductive coupling to charge an implanted battery, transcutaneous energy transfer by magnetic inductive coupling involves the placement of two coils positioned in close proximity to each other on opposite sides of the cutaneous boundary. The internal coil is integrated in the implanted device. The external coil is associated with an external power source and a current is induced in the internal coil through inductive coupling to charge the battery of the implanted device.

In an embodiment where low-power radio frequency is used to charge the battery, an external unit emits high-frequency electromagnetic waves which can charge an implanted device or recharge the local power storage.

In one embodiment, where mechanical waves such as ultrasound is used to charge the battery, this technique exploits the ability of acoustic waves to penetrate deeper in the body tissue without being significantly attenuated. In one specific embodiment, the power supply includes an external control unit (CU) and the implanted medical device (IMD) such as the detector unit which transfer energy and data by an acoustic link. An electrical signal can be transformed into an acoustic wave by a piezoelectric transducer contained in a control unit external to the body. Then this acoustic wave propagates in the direction of the power supply. Another piezoelectric transducer is integrated inside the implanted device and receives the acoustic wave coming from the control unit and converts it to electrical energy to be stored in the internal battery (Peisino 2013). Ultrasonic energy transfer through small transducers and to deep implanted devices proved more efficient than magnetic induction and radiofrequency waves and can be used for energization and communication in active implanted medical devices.

### Transmitter

In one embodiment the detector device includes a transmitter that transmits biomolecule sequence and identity information to a receiver-relay unit. In one embodiment the transmitter is an in vivo ultrasonic transponder system for biomedical applications. Ultrasonic telemetry provide for communication between one or several sensors or stimulators deeply implanted in the human body (the transponders) and a control unit which is used for both wirelessly recharging the implanted devices and transmitting the received information outside the body. The ultrasponder technology is a telemetry technique based on the backscattering principle to ensure efficient data communication through acoustic waves from the implanted transponder to the external control unit. In the backscattering technique, the reader is the control unit and the tag is the transponder. The communication is unidirectional, the control unit provides the energy for the carrier wave, which illuminates the transponder. The transponder modulates this carrier by impedance modulation and scatters it back to the control unit. Finally the control unit receives the backscattered wave and demodulates the signal. Demodulation occurs only at CU level and modulation only at transponder level. This reduces power consumption on transponder, as the implant will not need to demodulate instructions coming from the control unit. The high flexibility and modularity of the transponder is adaptable for the sensors used in the detection unit. Wireless communication can occur through acoustic waves from the control unit to the transponder. The ultrasponder implantable transponder contains an energy exchanger which coverts acoustic energy into electrical energy, a small local energy storage, a control and processing chip, and a sensor or actuator, all enclosed in a miniaturized biocompatible casing, hermetically sealed. The device may be equipped with an alarm function to facilitate critical care monitoring in certain applications. The ultrasponder device will also incorporate a dedicated external control unit, capable of energizing the transponder or transponder network and receiving information directly from the implanted transponders.

### Biological Fluids

The detector device is intended for use in screening biological fluids in a subject. In one embodiment, the biological fluids are within, excreted by, or secreted by a subject. Examples of such biological fluids include but are not limited to blood, serum, plasma, lymph, perspiration, urine, tears, saliva, and any biological fluid that contains biomolecule targets that indicate the onset, presence or progression of disease are contemplated for use with the present detector device.

### Diseases

While the detector device described herein is particularly useful for detecting and monitoring cancer, the detector device may be modified appropriate for use in detecting and monitoring other diseases using the same principles. Such diseases may be grouped into three main categories: neoplastic disease, inflammatory disease, and degenerative disease.

Examples of diseases include, but are not limited to, metabolic diseases (e.g., obesity, cachexia, diabetes, anorexia, etc.), cardiovascular diseases (e.g., atherosclerosis, ischemia/reperfusion, hypertension, myocardial infarction, restenosis, cardiomyopathies, arterial inflammation, etc.), immunological disorders (e.g., chronic inflammatory diseases and disorders, such as Crohn's disease, inflammatory bowel disease, reactive arthritis, rheumatoid arthritis, osteoarthritis, including Lyme disease, insulin-dependent diabetes, organ-specific autoimmunity, including multiple sclerosis, Hashimoto's thyroiditis and Grave's disease, contact dermatitis, psoriasis, graft rejection, graft versus host disease, sarcoidosis, atopic conditions, such as asthma and allergy, including allergic rhinitis, gastrointestinal allergies, including food allergies, eosinophilia, conjunctivitis, glomerular nephritis, certain pathogen susceptibilities such as helminthic (e.g., leishmaniasis) and certain viral infections, including HIV, and bacterial infections, including tuberculosis and lepromatous leprosy, etc.), myopathies (e.g. polymyositis, muscular dystrophy, central core disease, centronuclear (myotubular) myopathy, myotonia congenita, nemaline myopathy, paramyotonia congenita, periodic paralysis, mitochondrial myopathies, etc.), nervous system disorders (e.g., neuropathies, Alzheimer's disease, Parkinson's disease, Huntington's disease, amyotropic lateral sclerosis, motor neuron disease, traumatic nerve injury, multiple sclerosis, acute disseminated encephalomyelitis, acute necrotizing hemorrhagic leukoencephalitis, dysmyelination disease, mitochondrial disease, migrainous disorder, bacterial infection, fungal infection, stroke, aging, dementia, peripheral nervous system diseases and mental disorders such as depression and schizophrenia, etc.), oncological disorders (e.g., leukemia, brain cancer, prostate cancer, liver cancer, ovarian cancer, stomach cancer, colorectal cancer, throat cancer, breast cancer, skin cancer, melanoma, lung cancer, sarcoma, cervical cancer, testicular cancer, bladder cancer, endocrine cancer, endometrial cancer, esophageal cancer, glioma, lymphoma, neuroblastoma, osteosarcoma, pancreatic cancer, pituitary cancer, renal cancer, and the like) and ophthalmic diseases (e.g. retinitis pigmentosum and macular degeneration). The term also includes disorders, which result from oxidative stress, inherited cancer syndromes, and other metabolic diseases.

### Disease markers

Suitable examples of prognostic targets may include enzymatic targets such as galactosyl transferase II, neuron specific enolase, proton ATPase-2, or acid phosphatase.

Suitable examples of hormone or hormone receptor targets may include human chorionic gonadotropin (HCG), adrenocorticotropic hormone, carcinoembryonic antigen (CEA), prostate-specific antigen (PSA), estrogen receptor, progesterone receptor, androgen receptor, gC1q-R/p33 complement receptor, IL-2 receptor, p75 neurotrophin receptor, PTH receptor, thyroid hormone receptor, or insulin receptor.

Suitable examples of lymphoid targets may include alpha-1-antichymotrypsin, alpha-1-antitrypsin, B cell target, bcl-2, bcl-6, B lymphocyte antigen 36 kD, BM1 (myeloid target), BM2 (myeloid target), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage target, muramidase (lysozyme), p80 anaplastic lymphoma kinase, plasma cell target, secretory leukocyte protease inhibitor, T cell antigen receptor (JOVI 1), T cell antigen receptor (JOVI 3), terminal deoxynucleotidyl transferase, or unclustered B cell target.

Suitable examples of tumour targets may include alpha fetoprotein, apolipoprotein D, BAG-1 (RAP46 protein), CA 15-3, CA19-9 (sialyl lewisa), CA50 (carcinoma associated mucin antigen), CA125 (ovarian cancer antigen), CA242 (tumour associated mucin antigen), chromogranin A, clusterin (apolipoprotein J), epithelial membrane antigen, epithelial-related antigen, epithelial specific antigen, gross cystic disease fluid protein-15, hepatocyte specific antigen, heregulin, human gastric mucin, human milk fat globule, MAGE-1, matrix metalloproteinases, melan A, melanoma target (HMB45), mesothelin, metallothionein, microphthalmia transcription factor (MITE), Muc-1 core glycoprotein. Muc-1 glycoprotein, Muc-2 glycoprotein, Muc-5AC glycoprotein, Muc-6 glycoprotein, myeloperoxidase, Myf-3 (Rhabdomyosarcoma target), Myf-4 (Rhabdomyosarcoma target), MyoD1 (Rhabdomyosarcoma target), myoglobin, nm23 protein, placental alkaline phosphatase, prealbumin, prostate specific antigen, prostatic acid phosphatase, prostatic inhibin peptide, PTEN, renal cell carcinoma target, small intestinal mucinous antigen, tetranectin, thyroid transcription factor-1, tissue inhibitor of matrix metalloproteinase 1, tissue inhibitor of matrix metalloproteinase 2, tyrosinase, tyrosinase-related protein-1, villin, or von Willebrand factor.

Suitable examples of cell cycle associated targets may include apoptosis protease activating factor-1, bcl-w, bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin B1, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, Mcl-1, minichromosome maintenance proteins, mismatch repair protein (MSH2), poly (ADP-Ribose) polymerase, proliferating cell nuclear antigen, p16 protein, p27 protein, p34cdc2, p57 protein (Kip2), p105 protein, Stat 1 alpha, topoisomerase I, topoisomerase II alpha, topoisomerase III alpha, or topoisomerase II beta.

Suitable examples of neural tissue and tumor targets may include alpha B crystallin, alpha-internexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma target, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S-100 protein, serotonin, SNAP-25, synapsin I, synaptophysin, tau, tryptophan hydroxylase, tyrosine hydroxylase, or ubiquitin.

Suitable examples of cluster differentiation targets may include CD1a, CD1b, CD1c, CD1d, CD1e, CD2, CD3delta, CD3epsilon, CD3gamma, CD4, CD5, CD6, CD7, CD8alpha, CD8beta, CD9, CD10, CD11a, CD11b, CD11c, CDw12, CD13, CD14, CD15, CD15s, CD16a, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDw108, CD109, CD114, CD115, CD116, CD117, CDw119, CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD164, CD165, CD166, and TCR-zeta.

Other suitable prognostic targets may include centromere protein-F (CENP-F), giantin, involucrin, lamin A&C(XB 10), LAP-70, mucin, nuclear pore complex proteins, p180 lamellar body protein, ran, r, cathepsin D, Ps2 protein, Her2-neu, P53, S100, epithelial target antigen (EMA), TdT, MB2, MB3, PCNA, or Ki67.

For monitoring onset or progression of proliferative diseases such as cancer, a number of tumor markers are known and can be detected using the detector device described herein. Tumor markers include, but are not limited to, epidermal growth factor receptor-related protein c-erbB2 (Dsouza, B. et al. (1993) Oncogene. 8: 1797-1806), the glycoprotein MUC1 (Batra, S. K. et al. (1992) Int. J. Pancreatology. 12: 271-283) and the signal transduction/cell cycle regulatory proteins Myc (Blackwood, E. M. et al. (1994) Molecular Biology of the Cell 5: 597-609), p53 (Matlashewski, G. et al. (1984) EMBO J. 3: 3257-3262; Wolf, D. et al. (1985) Mel. Cell. Biol. 5: 1887-1893) and ras (or Ras) (Capella, G. et al. (1991) Environ Health Perspectives. 93: 125-131), including the viral oncogenic forms of ras which can be used as antigens to detect anti-ras autoantibodies, and also BRCA1 (Scully, R. et al. (1997) PNAS 94: 5605-10), BRCA2 (Sharan, S. K. et al. (1997) Nature. 386: 804-810), APC (Su, L. K. et al. (1993) Cancer Res. 53: 2728-2731; Munemitsu, S. et al. (1995) PNAS 92: 3046-50), CA125 (Nouwen, E. J. et al. (1990) Differentiation. 45: 192-8) and PSA (Rosenberg, R. S. et al. (1998) Biochem Biophys Res Commun. 248: 935-939), p53, "Melanoma Inhibitory Activity" MIA, HTZ-19 (Bogdahn et al., Cancer Res. 1989; 49: 5358-5363), S-100B (Bouwhuis et al., Eur J Cancer. 2011 Feb;47(3):361-8.

Tumour markers detectable directly from genomic sampling or sequencing of detectable cell-free nucleic acids may comprise the list of genetic / genomic corollaries of those listed above, and particularly including genes for, and mutations in, particularly such markers as KRAS, BRAF, EGFR (Epidermal Growth Factor Receptor), as well as ABL1, BTK, CTNNB1, FGF23, IL7R, MLH1, PDGFRA, SMO, AKT1, CARD11, DAXX, FGF3, INHBA, KMT2A (MLL), PDGFRB, SOCS1, AKT2, CBFB, DDR2, FGF4, IRF4, KMT2D (MLL2), PDK1, SOX10, AKT3, CBL, DNMT3A, FGF6, IRS2, MPL, PIK3CA, SOX2, ALK, CCND1, DOT1L, FGFR1, JAK1, MRE11A, PIK3CG, SPEN,APC, CCND2, EGFR, FGFR2, JAK2, MSH2, PIK3R1, SPOP, AR, CCND3, EMSY (C11orf30), FGFR3, JAK3, MSH6, PIK3R2, SRC, ARAF, CCNE1, EP300, FGFR4, JUN, MTOR, PPP2R1A, STAG2, ARFRP1, CD79A, EPHA3, FLT1, KAT6A (MYST3), MUTYH, PRDM1, STAT4, ARID1A, CD79B, EPHA5, FLT3, KDM5A, MYC, PRKAR1A, STK11, ARID2, CDC73, EPHB1, FLT4, KDM5C, MYCL1, PRKDC, SUFU, ASXL1, CDH1, ERBB2, FOXL2, KDM6A, MYCN, PTCH1, TET2, ATM, CDK12, ERBB3, GATA1, KDR, MYD88, PTEN, TGFBR2, ATR, CDK4, ERBB4, GATA2, KEAP1, NF1, PTPN11, TNFAIP3, ATRX, CDK6, ERG, GATA3, KIT, NF2, RAD50, TNFRSF14, AURKA, CDK8, ESR1, GID4 (C17orf39), KLHL6, NFE2L2, RAD51, TOP1, AURKB, CDKN1B, EZH2, GNA11, KRAS, NFKBIA, RAF1, TP53, AXL, CDKN2A, FAM123B (WTX), GNA13, LRP1B, NKX2-1, RARA, TSC1, BAP1, CDKN2B, FAM46C, GNAQ, MAP2K1, NOTCH1, RB1, TSC2, BARD1, CDKN2C, FANCA, GNAS, MAP2K2, NOTCH2, RET, TSHR, BCL2, CEBPA, FANCC, GPR124, MAP2K4, NPM1, RICTOR, VHL, BCL2L2, CHEK1, FANCD2, GRIN2A, MAP3K1, NRAS, RNF43, WISP3, BCL6, CHEK2, FANCE, GSK3B, MCL1, NTRK1, RPTOR, WT1, BCOR, CIC, FANCF, HGF, MDM2, NTRK2, RUNX1, XPO1, BCORL1, CREBBP, FANCG, HRAS, MDM4, NTRK3, SETD2, ZNF217, BLM, CRKL, FANCL, IDH1, MED12, NUP93, SF3B1, ZNF703, BRAF, CRLF2, FBXW7, IDH2, MEF2B, PAK3, SMAD2, BRCA1, CSF1R, FGF10, IGF1R, MEN1, PALB2, SMAD4, BRCA2, CTCF, FGF14, IKBKE, MET, PAX5, SMARCA4, BRIP1, CTNNA1, FGF19, IKZF1, MITF, PBRM1, SMARCB1, BCR, ETV4, ETV5, ETV6, EWSR1, ROS1, TMPRSS2, ACTB, AMER1, APH1A, ARHGAP26, ASMTL, AXIN1, B2M, BCL10, BCL11B, BCL7A, BIRC3, BRD4, BRSK1, BTG2, BTLA, CAD, CCT6B, CD22, CD274, CD36, CD58, CD70, CHD2, CIITA, CKS1B, CPS1, CSF3R, CUX1, CXCR4, DDX3X, DNM2, DTX1, DUSP2, DUSP9, EBF1, ECT2L, EED, ELP2, EPHA7, ETS1, EXOSC6, FAF1, FAS, FBXO11, FBXO31, FHIT, FLCN, FLYWCH1, FOXO1, FOXO3, FOXP1, FRS2, GADD45B, GNA12, GTSE1, HDAC1, HDAC4, HDAC7M, HIST1H1C, HIST1H1D, HIST1H1E, HIST1H2AC, HIST1H2AG, HIST1H2AL, HIST1H2AM, HIST1H2BC, HIST1H2BJ, HIST1H2BK, HIST1H2BO, HIST1H3B, HNF1A, HSP90AA1, ICK, ID3, IKZF2, IKZF3, INPP4B, INPP5D, IRF1, IRF8, JARID2, KDM2B, KDM4C, KMT2C, LEF1, LRRK2, MAF, MAFB, MAGED1, MALT1, MAP3K14, MAP3K6, MAP3K7, MAPK1, MEF2C, MIB1, MKI67, MSH3, MYO18A, NCOR2, NCSTN, NOD1, NT5C2, NUP98, P2RY8, PAG1, PASK, PC, PCBP1, PCLO, PDCD1, PDCD11, PDCD1LG2, PDGFRB, PHF6, PIM1, PLCG2, POT1, PRSS8, PTPN2, PTPN6, PTPRO, RAD21, RASGEF1A, RELN, RHOA, S1PR2, SDHA, SDHB, SDHC, SDHD, SERP2, SEYBP1, SGK1, SMARCA1, SMC1A, SMC3, SOCS2, SOCS3, SRSF2, STAT3, STAT5A, STAT5B, STAT6, SUZ12, TAF1, TBL1XR1, TCF3, TCL1A, TLL2, TMEM30, TMSL3, TNFRSF11A, TNFRSF17, TP63, TRAF2, TRAF3, TRAF5, TUSC3, TYK2, U2AF1, U2AF2, WDR90, WHSC1, XBP1, YY1AP1, ZMYM3, ZNF24 and ZRSR2.

Other genes of interest which have oncogenic potential include USP17L2 (DUB3), BRF1, MTA1, and JAG2.

### EXAMPLES

### EXAMPLE 1: Perpetual Monitoring Of Tumor Transcripts With Implanted Detector Array

A detection device is implanted in communication with the circulation of an individual such that blood flow passes through a nanopore array within the detection device. The nanopore array is configured for single strand sequencing of DNA in a format similar to the MinION (Oxford Nanotechnologies), with the added features of being substantially miniaturized to be implanted into a human circulation, and containing a wireless transmitter configured to transmit sequence data to a receiver located in close proximity to the human subject in order to receive the transmitted data signals from the detector device. The implanted detector device also contains an inductive power supply similar to those found in cardiac pacemakers all hermetically sealed except for the nanopore array which is permitted access to the circulation.

Circulating DNA in the human subject is detected via the nanopore array detector and tumor transcripts are screened for tumor markers including epidermal growth factor receptor-related protein c-erbB2 (Dsouza, B. et al. (1993) Oncogene. 8: 1797-1806), the glycoprotein MUC1 (Batra, S. K. et al. (1992) Int. J. Pancreatology. 12: 271-283) and the signal transduction/cell cycle regulatory proteins Myc (Blackwood, E. M. et al. (1994) Molecular Biology of the Cell 5: 597-609), p53 (Matlashewski, G. et al. (1984) EMBO J. 3: 3257-3262; Wolf, D. et al. (1985) Mel. Cell. Biol. 5: 1887-1893) and ras (or Ras) (Capella, G. et al. (1991) Environ Health Perspectives. 93: 125-131), including the viral oncogenic forms of ras which can be used as antigens to detect anti-ras autoantibodies, and also BRCA1 (Scully, R. et al. (1997) PNAS 94: 5605-10), BRCA2 (Sharan, S. K. et al. (1997) Nature. 386: 804-810), APC (Su, L. K. et al. (1993) Cancer Res. 53: 2728-2731; Munemitsu, S. et al. (1995) PNAS 92: 3046-50), CA125 (Nouwen, E. J. et al. (1990) Differentiation. 45: 192-8) and PSA (Rosenberg, R. S. et al. (1998) Biochem Biophys Res Commun. 248: 935-939) or p53, and epigenetic alternations (Gormally et al., Mutat Res 635(2-3):105-117 (2007)). Since tumor DNA is more plentiful in the circulation, the detector will detect tumor-specific DNA sequences, and changes in normally expressed transcripts that are associated with, and therefore indicate the presence of proliferative disease.

DNA sequencing data from the detector is transmitted wirelessly to a receiver located in close proximity to the human subject. The receiver may be worn on the person to receive the DNA sequencing data that is transmitted from the nanopore array detector. The receiver may optionally be incorporated as part of a patient's watch, cellular telephone, smart glasses, tablet, or personal accessory so long as the receiver device adequately communicates with the detector to receive the DNA sequence information. The receiver stores DNA sequence information in data files and then relays the data files wirelessly or via cable to a processor for analysis.

The processor analyzes the sequence information using multiple comparisons and algorithms to constantly monitor sequence information compared to normal human subject controls where no cancer is present.

If tumor-specific or cancer-specific DNA sequences are detected, or if tumor-associated alterations in transcript concentration is detected, the processor sends a signal to the receiver and optionally to the human subject's healthcare provider to notify the detection of tumor-associated sequences.

### EXAMPLE 2: Detection of Melanoma With an On-Board Detector Array

A portable detector is worn on an individual and sweat from the individual is monitored for the presence of melanoma markers S-100B and/or melanoma-inhibiting activity (MIA).

Protein sequencing data from the detector is transmitted wirelessly to a receiver in the individual's smart phone located in close proximity. The receiver relays the data files wirelessly or via cable to a processor for analysis.

The processor analyzes the sequence information using multiple comparisons and algorithms to the sequence information compared to normal human subject controls where no cancer is present. If a significant difference in S-100B or MIA exists compared to normal, healthy, non-cancer individuals, an alert will be transmitted to both the individual and their healthcare provider so that they may take immediate action.

### EXAMPLE 3: Perpetual Monitoring Of Metastatic Breast Cancer With Implanted Detector Array

A detection device is implanted in communication with the circulation of an individual such that blood flow passes through a nanopore array within the detection device. The nanopore array is configured for single strand sequencing of DNA in a format similar to the MinION (Oxford Nanotechnologies), with the added features of being substantially miniaturized to be implanted into a human circulation, and containing a wireless transmitter configured to transmit sequence data to a receiver located in close proximity to the human subject in order to receive the transmitted data signals from the detector device. The implanted detector device also contains an inductive power supply similar to those found in cardiac pacemakers all hermetically sealed except for the nanopore array which is permitted access to the circulation.

Circulating DNA in the human subject is detected via the nanopore array detector and tumor transcripts are screened for tumor markers CA 15-3 ; mutations in PIK3CA and TP53 (Dawson et al. N Engl J Med 368:1199-209 (2013); Benesova et al., Anal Biochem 433:227-234 (2013); and SNP/copy number variation (Shaw et al., Genome Res 22:220-223 (2012). Since tumor DNA is more plentiful in the circulation, the detector will detect tumor-specific DNA sequences, and changes in normally expressed transcripts that are associated with, and therefore indicate the presence of proliferative disease.

DNA sequencing data from the detector is transmitted wirelessly to a receiver located in close proximity to the human subject. The receiver may be worn on the person to receive the DNA sequencing data that is transmitted from the nanopore array detector. The receiver may optionally be incorporated as part of a patient's watch, cellular telephone, or personal accessory so long as the receiver device adequately communicates with the detector to receive the DNA sequence information. The receiver stores DNA sequence information in data files and then relays the data files wirelessly or via cable to a processor for analysis.

The processor analyzes the sequence information using multiple comparisons and algorithms to constantly monitor sequence information compared to normal human subject controls where no cancer is present.

If tumor-specific or cancer-specific DNA sequences are detected, or if tumor-associated alterations in transcript concentration is detected, the processor sends a signal to the receiver and to the human subject's healthcare provider to notify the detection of tumor-associated sequences.

### EXAMPLE 4: Monitoring Of Treatment for Tuberculosis With Implanted Detector Array

A detection unit device is implanted in fluid communication with the circulation of an individual such that blood flow passes through a nanopore array within the detection device. The detector unit is configured for blood cell transcriptome sequencing using nanopore arrays, with the added features of being substantially miniaturized to be implanted into a human circulation, and containing a wireless transmitter configured to transmit sequence data to a receiver located in close proximity to the human subject in order to receive the transmitted data signals from the detector device. The implanted detector device also contains an inductive power supply similar to those found in cardiac pacemakers all hermetically sealed except for the nanopore array which is permitted access to the circulation.

Circulating DNA in the human subject is detected via the nanopore array detector and blood transcriptome transcripts are screened for efficacy of tuberculosis treatment. Changes in the blood transcriptome can be detected within 2 weeks of tuberculosis treatment. (Bloom et al., PLOS ONE: 7(10):1-13 (2012))

DNA sequencing data from the detector is transmitted wirelessly to a receiver located in close proximity to the human subject. The receiver may be worn on the person to receive the DNA sequencing data that is transmitted from the nanopore array detector. The receiver may optionally be incorporated as part of a patient's watch, cellular telephone, or personal accessory so long as the receiver device adequately communicates with the detector to receive the DNA sequence information. The receiver stores DNA sequence information in data files and then relays the data files wirelessly or via cable to a processor for analysis.

The processor analyzes the sequence information using multiple comparisons and algorithms to constantly monitor sequence information compared to normal human subject controls where tuberculosis treatment is effective.

As treatment progresses, the processor sends a signal to the receiver and to the human subject's healthcare provider to notify of progress of treatment and changes in the blood transcriptome that indicate efficacy of treatment.

### EXAMPLE 5: Monitoring Of Hepatocellular Carcinoma With Implanted Detector Array

A detection device is implanted in communication with the circulation of an individual such that blood flow passes through a nanopore array within the detection device. The nanopore array is configured for single strand sequencing of DNA in a format similar to the MinION (Oxford Nanopore Technologies), with the added features of being substantially miniaturized to be implanted into a human circulation, and containing a wireless transmitter configured to transmit sequence data to a receiver located in close proximity to the human subject in order to receive the transmitted data signals from the detector device. The implanted detector device also contains an inductive power supply similar to those found in cardiac pacemakers all hermetically sealed except for the nanopore array which is permitted access to the circulation. Circulating DNA in the human subject is detected via the nanopore array detector and tumor transcripts are screened for tumor associated marker copy number (Chen et al., Clin Chem 59(1):211-224 (2013)). Since tumor DNA is more plentiful in the circulation, the detector will detect tumor-specific DNA sequences, and changes in normally expressed transcripts that are associated with, and therefore indicate the presence of hepatocellular carcinoma.

DNA sequencing data from the detector is transmitted wirelessly to a receiver located in close proximity to the human subject. The receiver may be worn on the person to receive the DNA sequencing data that is transmitted from the nanopore array detector. The receiver may optionally be incorporated as part of a patient's watch, cellular telephone, or personal accessory so long as the receiver device adequately communicates with the detector to receive the DNA sequence information. The receiver stores DNA sequence information in data files and then relays the data files wirelessly or via cable to a processor for analysis.

The processor analyzes the sequence information using multiple comparisons and algorithms to constantly monitor sequence information compared to normal human subject controls where no cancer is present.

If tumor-associated copy number increases are detected, or if tumor-associated alterations in transcript concentration are detected, the processor sends a signal to the receiver and to the human subject's healthcare provider to notify the detection of the presence of hepatocellular carcinoma.

### EXAMPLE 6: Monitoring Of Pancreatic Carcinoma With Implanted Detector Array

A detection device is implanted in communication with the circulation of an individual such that blood flow passes through a sequencing array within the detection device. The sequencing array is configured for single strand sequencing of DNA, with the added features of being substantially miniaturized to be implanted into a human circulation, and containing a wireless transmitter configured to transmit sequence data to a receiver located in close proximity to the human subject in order to receive the transmitted data signals from the detector device. The implanted detector device also contains an inductive power supply similar to those found in cardiac pacemakers all hermetically sealed except for the sequencing array which is permitted access to the circulation.

Circulating DNA in the human subject is captured and sequenced for detection via the sequencing array detector and tumor transcripts are screened for tumor associated marker copy number (Chen et al., Clin Chem 59(1):211-224 (2013)). Since tumor DNA is more plentiful in the circulation, the detector will detect tumor-specific DNA sequences, and changes in normally expressed transcripts that are associated with, and therefore indicate the presence of hepatocellular pancreatic.

DNA sequencing data from the detector is transmitted wirelessly to a receiver located in close proximity to the human subject. The receiver may be worn on the person to receive the DNA sequencing data that is transmitted from the sequencing array detector. The receiver may optionally be incorporated as part of a patient's watch, cellular telephone, or personal accessory so long as the receiver device adequately communicates with the detector to receive the DNA sequence information. The receiver stores DNA sequence information in data files and then relays the data files wirelessly or via cable to a processor for analysis.

The processor analyzes the sequence information using multiple comparisons and algorithms to constantly monitor sequence information compared to normal human subject controls where no cancer is present.

If tumor-associated copy number increases are detected, or if tumor-associated alterations in transcript concentration are detected, the processor sends a signal to the receiver and to the human subject's healthcare provider to notify the detection of the presence of pancreatic carcinoma.

### EXAMPLE 7: Monitoring Of Serum Cholesterol Levels With Implanted Detector Unit

A detection device is implanted in communication with the circulation of an individual such that blood flow passes through a miniaturized lab-on-a-chip unit within the detection device. The lab-on-a-chip unit is configured for detection and monitoring of serum cholesterol concentration over time, with the added features of being substantially miniaturized to be implanted into a human circulation, and containing a wireless transmitter configured to transmit data regarding serum cholesterol levels to a receiver located in close proximity to the human subject in order to receive the transmitted data signals from the detector device. The implanted detector device also contains an inductive power supply similar to those found in cardiac pacemakers all hermetically sealed except for the lab-on-a-chip unit which is permitted access to the circulation.

Serum cholesterol in the human subject is detected via the lab-on-a-chip unit detector. A baseline cholesterol level is established and serum concentrations are monitored for a defined period of time afterwards.

Cholesterol concentration data from the detector is transmitted wirelessly to a receiver located in close proximity to the human subject. The receiver may be worn on the person to receive the data that is transmitted from the lab-on-a-chip unit detector. The receiver may optionally be incorporated as part of a patient's watch, cellular telephone, or personal accessory so long as the receiver device adequately communicates with the detector to receive the cholesterol level information. The receiver stores cholesterol information in data files and then relays the data files wirelessly or via cable to a processor for analysis.

The processor analyzes the cholesterol level information using multiple comparisons and algorithms to constantly monitor cholesterol level information compared to normal human subject controls having normal cholesterol levels.

Periodically, the processor sends a signal to the receiver and to the human subject's healthcare provider to notify the serum cholesterol concentration.

## Claims

1. A detector device for detecting the presence of one or more biomolecule markers selected from DNA or RNA in a biological fluid in a subject comprising:
a) detection means in fluid communication with a biological fluid capable of obtaining information regarding the identity and concentration of a biomolecule, wherein the detector includes a sequencing module, wherein the sequencing module is a nanopore detection device comprising one or more nanopores for detecting and sequencing DNA or RNA,
b) a power supply, and
c) means to transmit the information to a receiver-relay unit.

2. The detector device of claim 1 wherein the nanopores are biological, solid-state or hybrid pores that permit the detection and sequencing of nucleic acid to provide real-time DNA sequencing of nucleic acids in a biological fluid.

3. The detector device of claim 2 wherein the solid-state nanopores are made of synthetic materials such as silicon nitride or graphene.

4. The detector device of any one of claims 1 to 3 wherein the detector comprises an array of nanopores for multiplex evaluation of biomolecules.

5. The detector device of any one of claims 1 to 4 wherein the detector is implantable located at a surgically created arterio-venous fistula or shunt, between the arterial and venous systems.

6. The detector device of any one of claims 1 to 5 wherein the detector includes an array of nanopores for multiplex detection of nucleic acid bases and linear nucleic acid sequencing.

7. The detector device of any one of claims 1 to 6 wherein the detector is arranged in such a manner as to permit flow of a biological fluid through the nanopore array to permit detection and evaluation of the biomolecules without substantially affecting the flow of the biological fluid on its proper course.

8. The detector device of any one of claims 1 to 7 wherein the array of nucleic acid base-detecting nanopores permits sequencing of nucleic acid present in the biological fluid to evaluate the identity of, and characteristics of the nucleic acid that indicates disease.

9. The detector device of any one of claims 1 to 8 wherein a biological protective matrix layer covers the surface of the detector device.

10. The detector device of any one of claims 1 to 9 wherein data transmission to the receiver-relay is continuous or periodic and can be controlled by receiving instruction signals from the receiver-relay.

11. The detector device of any one of claims 1 to 10 wherein the device is implantable or wearable in a host.

12. The detector device of claim 11 wherein the device is implantable in a blood vessel or a blood vessel bypass graft.

13. The detector device of any one of claims 1 to 12 wherein the device is in fluid communication with the lymphatic system.

14. The detector device of any one of claims 1 to 13 wherein the device further comprises means for monitoring, and a feedback loop to control directly, and/or signal the patency of the feeding vessel/system and biological fluid flow to the device and resultant reliable access to biomolecules.

## Patentansprüche

1. Erkennungsvorrichtung zum Erkennen des Vorhandenseins von einem oder mehreren Biomolekülmarkern, die aus der DNS oder RNS in einem biologischen Fluid in einem Patienten ausgewählt sind, umfassend:
a) Erkennungsmittel in Fluidkommunikation mit einem biologischen Fluid, das in der Lage ist, Informationen bezogen auf die Identität und Konzentration eines Biomoleküls zu erhalten, wobei der Erkenner ein Sequenzierungsmodul beinhaltet, wobei das Sequenzierungsmodul eine Nanoporenerkennungsvorrichtung ist, die eine oder mehrere Nanoporen zum Erkennen und Sequenzieren von DNS oder RNS umfasst,
b) eine Stromquelle und
c) Mittel zum Übertragen der Informationen an eine Empfängerrelaiseinheit,

2. Erkennungsvorrichtung nach Anspruch 1, wobei die Nanoporen biologische Festkörper- oder Hybridporen sind, welche die Erkennung und Sequenzierung von Nukleinsäure ermöglichen, um eine Echtzeit-DNS-Sequenzierung von Nukleinsäuren in einem biologischen Fluid bereitzustellen.

3. Erkennungsvorrichtung nach Anspruch 2, wobei die Festkörpernanophoren aus synthetischen Materialien hergestellt sind, wie etwa Siliziumnitrit oder Graphen.

4. Erkennungsvorrichtung nach einem der Ansprüche 1 bis 3, wobei der Erkenner eine Anordnung von Nanoporen für eine Multiplex-Bewertung von Biomolekülen umfasst.

5. Erkennungsvorrichtung nach einem der Ansprüche 1 bis 4, wobei der Erkenner implantierbar an einer bzw. einem chirurgisch erzeugten arterienvenösen Fistel oder Shunt zwischen dem Arterien- und Venensystem angeordnet ist.

6. Erkennungsvorrichtung nach einem der Ansprüche 1 bis 5, wobei der Erkenner eine Anordnung von Nanoporen zur Multiplex-Erkennung von Nukleinsäurebasen und zur linearen Nukleinsäuresequenzierung beinhaltet.

7. Erkennungsvorrichtung nach einem der Ansprüche 1 bis 6, wobei der Erkenner in einer solchen Weise angeordnet ist, dass ein Strom von einem biologischem Fluid durch die Nanoporenanordnung ermöglicht wird, um eine Erkennung und Bewertung der Biomoleküle zu ermöglichen, ohne den Strom des biologischen Fluids im Wesentlichen in seiner angemessenen Bahn zu beeinflussen.

8. Erkennungsvorrichtung nach einem der Ansprüche 1 bis 7, wobei die Anordnung von nukleinsäurebasenerkennenden Nanoporen ein Sequenzieren von Nukleinsäure ermöglicht, die in dem biologischen Fluid vorhanden ist, um die Identität und Eigenschaften der Nukleinsäure zu bewerten, die eine Krankheit anzeigt.

9. Erkennungsvorrichtung nach einem der Ansprüche 1 bis 8, wobei eine biologische, schützende Matrixschicht die Oberfläche der Erkennungsvorrichtung bedeckt.

10. Erkennungsvorrichtung nach einem der Ansprüche 1 bis 9, wobei die Datenübertragung an das Empfängerrelais durchgehend oder in regelmäßigen Abständen erfolgt und durch Empfangen von Anweisungssignalen von dem Empfängerrelais gesteuert werden kann.

11. Erkennungsvorrichtung nach einem der Ansprüche 1 bis 10, wobei die Vorrichtung in einem Wirt implantiert oder getragen werden kann.

12. Erkennungsvorrichtung nach Anspruch 11, wobei die Vorrichtung in einem Blutgefäß oder einem Blutgefäßumgehungsimplantat implantiert werden kann.

13. Erkennungsvorrichtung nach einem der Ansprüche 1 bis 12, wobei die Vorrichtung mit dem Lymphsystem in Fluidkommunikation steht.

14. Erkennungsvorrichtung nach einem der Ansprüche 1 bis 13, wobei die Vorrichtung ferner Mittel zum Überwachen und eine Rückkopplungsschleife zum direkten Steuern und/oder Signalisieren der Durchgängigkeit des Zuführgefäßes/-systems und des biologischen Fluidstroms zu der Vorrichtung und dem resultierenden zuverlässigen Zugriff auf Biomoleküle umfasst.

## Revendications

1. Dispositif de détection pour détecter la présence d'un ou de plusieurs marqueurs biomoléculaires choisis par l'ADN ou l'ARN dans un fluide biologique chez un sujet comprenant :
a) la détection de moyens en communication fluidique avec un fluide biologique capable d'obtenir des informations concernant l'identité et la concentration d'une biomolécule, dans lequel le détecteur comprend un module de séquençage, dans lequel le module de séquençage est un dispositif de détection de nanopores comprenant un ou plusieurs nanopores pour la détection et le séquençage de l'ADN ou de l'ARN,
b) une alimentation électrique, et
c) des moyens pour transmettre les informations à une unité de relais récepteur.

2. Dispositif de détection selon la revendication 1, dans lequel les nanopores sont des pores biologiques, à l'état solide ou hybrides qui permettent la détection et le séquençage d'acide nucléique pour fournir un séquençage d'ADN en temps réel d'acides nucléiques dans un fluide biologique.

3. Dispositif de détection selon la revendication 2, dans lequel les nanopores à l'état solide sont constituées de matériaux synthétiques tels que le nitrure de silicium ou le graphène.

4. Dispositif de détection selon l'une quelconque des revendications 1 à 3, dans lequel le détecteur comprend un ensemble de nanopores pour une évaluation multiplex de biomolécules.

5. Dispositif de détection selon l'une quelconque des revendications 1 à 4, dans lequel le détecteur peut être implanté situé au niveau d'une fistule artério-veineuse ou d'un shunt artério-veineux créé de façon chirurgicale entre les systèmes artériel et veineux.

6. Dispositif de détection selon l'une quelconque des revendications 1 à 5, dans lequel le détecteur comprend un ensemble de nanopores pour une détection multiplex de bases d'acide nucléique et le séquençage d'acide nucléique linéaire.

7. Dispositif de détection selon l'une quelconque des revendications 1 à 6, dans lequel le détecteur est agencé de manière à permettre l'écoulement d'un fluide biologique à travers l'ensemble de nanopores pour permettre la détection et l'évaluation des biomolécules sans affecter sensiblement l'écoulement du fluide biologique sur sa propre voie.

8. Dispositif de détection selon l'une quelconque des revendications 1 à 7, dans lequel l'ensemble de nanopores de détection à base d'acide nucléique permet le séquençage d'acide nucléique présent dans le fluide biologique pour évaluer l'identité et les caractéristiques de l'acide nucléique qui indique une maladie.

9. Dispositif de détection selon l'une quelconque des revendications 1 à 8, dans lequel une couche de matrice de protection biologique recouvre la surface du dispositif de détection.

10. Dispositif de détection selon l'une quelconque des revendications 1 à 9, dans lequel la transmission de données au relais récepteur est continue ou périodique et peut être commandée en recevant des signaux d'instruction à partir du relais récepteur.

11. Dispositif de détection selon l'une quelconque des revendications 1 à 10, dans lequel le dispositif peut être implanté ou peut être porté chez un hôte.

12. Dispositif de détection selon la revendication 11, dans lequel le dispositif peut être implanté dans un vaisseau sanguin ou une greffe de pontage de vaisseau sanguin.

13. Dispositif de détection selon l'une quelconque des revendications 1 à 12, dans lequel le dispositif est en communication fluidique avec le système lymphatique.

14. Dispositif de détection selon l'une quelconque des revendications 1 à 13, dans lequel le dispositif comprend en outre des moyens pour surveiller et une boucle de rétroaction pour commander directement et/ou signaler la perméabilité du vaisseau/du système d'alimentation et l'écoulement de fluide biologique vers le dispositif et l'accès fiable résultant aux biomolécules.
